# EUROPÄISCHE PATENTSCHRIFT

(11) **EP 0 996 381 B1**
(45) Veröffentlichungstag und Bekanntmachung des Hinweises auf die Patenterteilung: **15.06.2005**
(21) Anmeldenummer: 98942557.4
(22) Anmeldetag: 14.07.1998
(51) Int. Cl.: A61B 18/00

(54) **EINRICHTUNG ZUR KOAGULATION BIOLOGISCHER GEWEBE MITTELS EINES IONIZIERBAREN GASES**
PREPARATION INSTRUMENTS
INSTRUMENT DE PREPARATION

(30) Priorität: 14.07.1997 DE 19730127
(43) Veröffentlichungstag der Anmeldung: 03.05.2000
(73) Patentinhaber: Erbe Elektromedizin GmbH, 72072 Tübingen (DE)
(72) Erfinder: FARIN, Günter, D-72070 Tübingen (DE); FISCHER, Klaus, D-72202 Nagold (DE)
(74) Vertreter: Bohnenberger, Johannes, Dr.
(86) Internationale Anmeldenummer: PCT/EP1998/004386
(87) Internationale Veröffentlichungsnummer: WO 1999/003406

(56) Entgegenhaltungen:
- EP-A- 0 688 536
- WO-A-92/05743
- WO-A-93/01758
- WO-A-96/24301
- DE-U- 9 409 884
- US-A- 5 449 356
- US-A- 5 571 137

## Beschreibung

Bei chirurgischen Operationen werden beim Durchtrennen von Gewebe Blutgefäße verletzt. Das austretende Blut nimmt zum einen dem Operateur die Sicht, zum anderen sind Blutverluste auf ein Minimum zu beschränken. Ein typisches Beispiel für eine Operation, bei der es häufig zu heftigen Blutungen kommt, ist die Tonsillektomie.

Zum Stillen von Blutungen sind aus der DE 41 39 029 A1 oder aus der DE 37 10 489 A1 Koagulationsvorrichtungen bekannt, bei welchen man in einer Edelgasatmosphäre einen HF-Strom durch Ionisierung des Edelgases bzw. Erzeugung eines Plasmas zwischen einer Elektrode und dem Gewebe fließen läßt. Durch die entstehende Wärme wird das Gewebe koaguliert. Eine solche Vorrichtung muß zusätzlich zum Präparationsinstrument verwendet werden.

Aus der WO 93/01758 ist eine Koagulationseinrichtung bekannt, bei welcher ein Präparationsinstrument, eine Nadel, eine Schere, eine Schlinge oder dergleichen bewegbar in einer Zuleitung für das Edelgas angeordnet ist, so daß der Operateur die durch ein Endoskop eingeführte Anordnung bewegen kann. Das Präparationsinstrument wird hierbei entweder ganz mechanisch wirkend, z. B. schneidend, oder aber durch Erzeugung eines direkten Stromflusses zwischen dem Instrument und dem Gewebe als elektrochirurgisches Instrument eingesetzt. Weiterhin kann das chirurgische Instrument bei der bekannten Anordnung als Elektrode zur Erzeugung eines Plasmas verwendet werden, wie dies bei den Gegenständen der eingangs genannten Druckschriften ebenfalls er Fall ist. Die Handhabung des bekannten Instrumentes beim Koagulieren ist sehr kritisch, da man unbedingt eine Berührung zwischen dem als Elektrode verwendeten Instrument und dem Gewebe vermeiden muss, um dieses nicht lokal zu zerstören. Weiterhin muss darauf geachtet werden, während des Präparierens nicht versehentlich den Koagulationsstrom einzuschalten, da dieser sonst direkt über das chirurgische Instrument auf das Gewebe wirkt. Dieses zeitliche Nacheinander sicherzustellen ist sehr aufwändig.

Aus der EP-A-0688536 ist ein Präparierinstrument bekannt, das als multifunktionales Instrument für die Ultraschall-Chirurgie ausgebildet ist. Das Arbeitsinstrument ist ein Ultraschall-Applikator. Von diesem getrennt angeordnet ist ein HF-Stromapplikator vorgesehen. Die verschiedenen Instrumente sind hierbei derart ausgebildet und angeordnet, dass sie in ihrer Relativposition zueinander verstellbar sind. Auf diese Weise kann der Operateur entweder den Ultraschall-Applikator (in seinem herausgeschobenen Zustand) verwenden oder aber Gewebe mittels eines HF-Stromes koagulieren, wobei dann der Ultraschall-Applikator zurückgezogen ist. Auch dieses Instrument ist kompliziert aufgebaut und erfordert zu seiner Bedienung höchste Konzentration und auch Übung.

Aus der US-A-5 449 356 ist ein elektrochirurgisches Gerät bekannt, das ein HF-chirurgisches Schneiden mittels einer Nadel oder Schlinge oder - alternativ - eine Plasma-Koagulation von Gewebe ermöglicht.

Der Erfindung liegt die Aufgabe zu Grunde, ein einfaches Präparierinstrument für chirurgische Operationen aufzuzeigen, das bei hoher Benutzerfreundlichkeit eine verbesserte Arbeitsweise ermöglicht.

Diese Aufgabe wird durch ein Präparierinstrument nach Patentanspruch 1 gelöst.

Ein wesentlicher Punkt der Erfindung liegt darin, dass die aufgezeigte Anordnung eine im Wesentlichen gleichzeitige Präparation und Koagulation mit ein und demselben Instrument ermöglicht. Man kann also praktisch während des Schneidens auftretende Blutungen sofort stillen bzw. Gewebe verschließen und zwar ohne einen erhöhten manuellen Aufwand. Dadurch werden die Operationsdauer wesentlich verkürzt, Blutverluste und die eingangs genannten Nachteile verringert oder gar verhindert und dennoch verringerte Anforderungen an den Operateur gestellt.

Hierbei wird das Arbeitsinstrument insgesamt aus einem Kunststoff, einem keramischen oder dergleichen isolierenden Material ausgebildet, was herstellungstechnisch relativ einfach ist. Alternativ, gegebenenfalls auch abschnittsweise zusätzlich kann das Arbeitsinstrument mit einem Kunststoff bzw. einem keramischen Material oder dergleichen überzogen werden, so daß kein elektrischer Strom über das Arbeitsinstrument zum Gewebe fließen kann, der den Fluß des Koagulationsstroms von der Elektrode zum Gewebe stören könnte.

Wichtig hierbei ist die Tatsache, daß bei der Plasma-Koagulation sozusagen selbsttätig nur diejenigen Gewebeabschnitte den "Eintritt" eines Stroms ermöglichen, die eine hinreichend hohe Leitfähigkeit aufweisen. Diese hinreichend hohe Leitfähigkeit ist gleichzeitig auch ein Maß dafür, wie "offen" bzw. feucht das Gewebe an seiner Oberfläche ist. Sobald eine hinreichend große Koagulationswirkung erzielt wurde, die Oberfläche des Gewebes also "trocken" ist, sucht sich der Koagulationsstrom einen anderen Weg bzw. wird unterbrochen, wenn die steuerbare HF-Quelle entsprechend (hinsichtlich Spannung und/oder Strom) eingestellt ist. Das isoliert aufgebaute Arbeitsinstrument kann somit diese "Selbstregelung" nicht stören. Vorzugsweise ist die Elektrodeneinrichtung, die einen Elektrodenhalter und eine Elektrode, insbesondere eine Drahtelektrode umfaßt, die nahe beim Arbeitsinstrument und vorzugsweise mit diesem zusammen fixiert ist. Dadurch ist ein einfacher und dennoch besonders gedrängter Aufbau möglich, so daß der Koagulationsstrom sehr direkt zu den Gewebeabschnitten fließen kann, an denen das Arbeitsinstrument gerade Veränderungen vornimmt.

Die Gaszuführungseinrichtung umfaßt eine Ausströmöffnung einer Gasleitung, die bei der Elektrodeneinrichtung oder diese umgebend nahe dem Arbeitsinstrument angeordnet ist. Auch diese Maßnahme führt zu einem besonders einfachen und gedrängten Aufbau.

Das Präparierinstrument ist dann besonders einfach, stabil und kleinbauend konstruierbar, wenn die Arbeitseinrichtung und der Elektrodenhalter im wesentlichen einstückig ausgebildet sind. Vorzugsweise ist hierbei auch gleichzeitig die Gasausströmöffnung mit der Arbeitseinrichtung einstückig ausgebildet.

Weiterhin ist es von Vorteil, wenn die Arbeitseinrichtung und mindestens ein distaler Endabschnitt der Halteeinrichtung im wesentlichen einstückig ausgebildet sind.

Die Elektrodeneinrichtung wird vorzugsweise derart ausgebildet und angeordnet, daß ein direkter elektrischer Kontakt zwischen der Elektrodeneinrichtung und dem Gewebe verhindert wird. Dies kann beispielsweise bei einer in der Gasausströmöffnung vorgesehenen Elektrode dadurch erreicht werden, daß diese etwas zurückgesetzt von der Öffnung angeordnet ist. Weiterhin ist es auch möglich, die Elektrode selbst mit einem Schutzkorb oder dergleichen zu umgeben, der einen direkten Kontakt zum Gewebe verhindert.

Der Edelgas-Strom sollte so "sanft" sein, daß er keine mechanische Wirkung entfaltet und auch nur eine sehr niedrige Gasmenge verbraucht wird, die bei verschiedenen Anwendungsorten sehr nachteilig wäre. Um nun dennoch ohne Zusatzinstrumente Flüssigkeit, Gewebestücke oder andere Festkörper von der Gewebeoberfläche entfernen zu können, insbesondere um das Operationsfeld freizumachen und um eine bessere Koagulationswirkung erzielen zu können, ist es möglich, zusätzlich (gegebenenfalls auch mit den zuvor beschriebenen Einrichtungen einstückig ausgebildet) weitere Einrichtungen wie Saug-, Blas- oder Spüleinrichtungen vorzusehen. Es ist beispielsweise möglich, zusätzlich zur Gasleitung zum Zuführen von ionisierbarem (Edel-)Gas zur Erzielung des Koagulationsstroms eine Gasdüse vorzusehen, die Flüssigkeit fortbläst. Ein gleichzeitiges Absaugen z. B. durch einen weiteren Kanal ist ebenfalls möglich. Selbstverständlich können mehrere Kanäle alternativ verschiedene Funktionen, z. B. eine Spülfunktion und eine Saugfunktion erfüllen.

Weiterhin ist es möglich, weitere zusätzliche elektrochirurgische Instrumente, wie z. B. eine Nadel-, Kugel- oder Flachelektrode vorzusehen, oder aber eine Drahtschlinge, um besondere Funktionen (z. B. Polypektomie) durchzuführen.

Vorzugsweise ist die Halteeinrichtung auswechselbar unter gleichzeitiger An- und Abkoppelung von Gas- und Stromzufuhr ausgebildet, so daß man unter Beibehaltung der Koagulationsbedingungen (bezüglich Gas- und Stromfluß) sehr schnell die Arbeitsinstrumente wechseln kann.

Um möglichst optimale Koagulationsbedingungen sicherzustellen, ist es von Vorteil, wenn Reinigungseinrichtungen zum Freihalten der Elektrodeneinrichtung von Flüssigkeit oder dergleichen verschmutzenden bzw. den Stromfluß ändernden Substanzen vorgesehen sind. Insbesondere dann, wenn die Elektrodeneinrichtung innerhalb der Gasausströmöffnung vorgesehen ist, kann man die Elektrode freihalten, indem ein im wesentlichen ständiger (sehr geringer) Gasstrom sichergestellt wird, der die Elektrodeneinrichtung umspült.

Bevorzugte Ausführungsformen der Erfindung ergeben sich aus den Unteransprüchen.

Nachfolgend wir die Erfindung anhand von Abbildungen bevorzugter Ausführungsformen näher erläutert.

Hierbei zeigen:
- Fig. 1: eine Ausführungsform der Erfindung in Seitenansicht,
- Fig. 2 bis 4: verschiedene Varianten von Arbeitsinstrument-Köpfen,
- Fig. 5: eine perspektivische Darstellung eines hakenförmigen Arbeitsinstrumentes,
- Fig. 6: einen Längsschnitt durch einen Endabschnitt des Instrumentes nach Fig. 5,

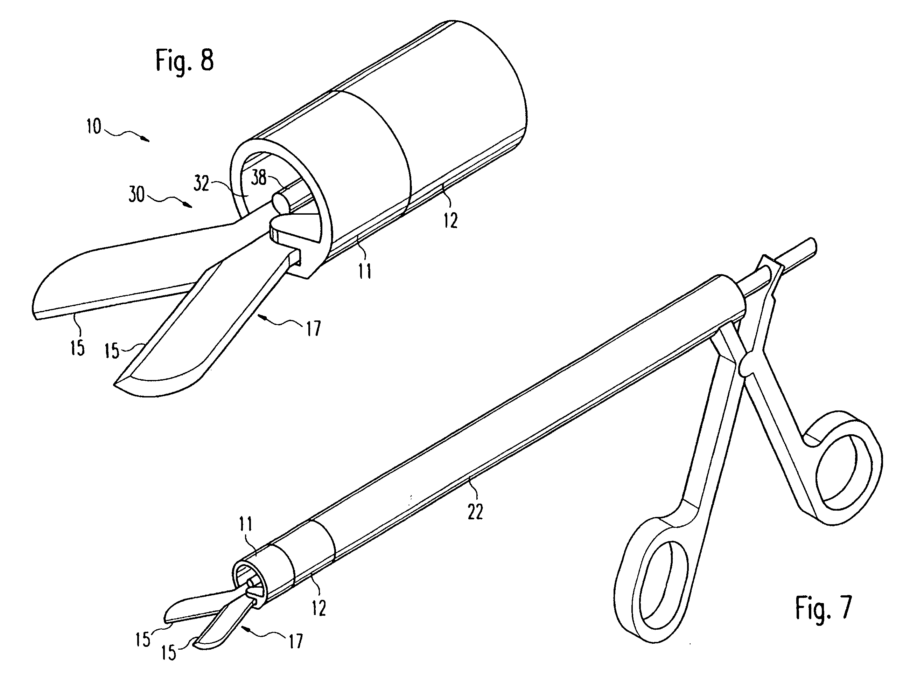

- Fig. 7: eine perspektivische Darstellung einer weiteren Ausführungsform eines Arbeitsinstrumentes,
- Fig. 8: eine Detailansicht des Endabschnittes des Instrumentes nach Fig. 7 und
- Fig. 9: eine perspektivische Darstellung eines weiteren Arbeitsinstrumentes.

In der nachfolgenden Beschreibung werden für gleiche und gleichwirkende Teile dieselben Bezugsziffern verwendet.

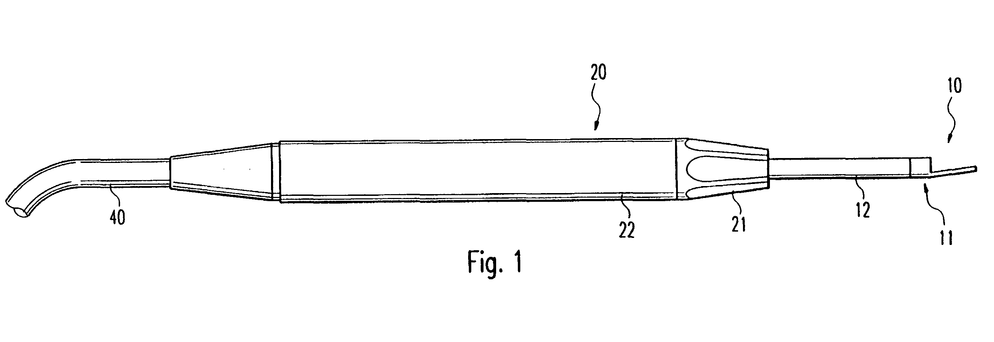

In Fig. 1 ist in einer Seitenansicht eine Ausführungsform eines Präparierinstruments für chirurgische Operationen gezeigt, das an seinem Vorderende ein Arbeitsinstrument 10 aufweist, das über einen Schaft 12 in einem Befestigungskonus 21 eines Handgriffs 22 einer Halteeinrichtung 20 befestigt ist. In ein hinteres Ende der Halteeinrichtung 20 ist ein Anschlußschlauch 40 geführt.

Das Arbeitsinstrument 10 umfaßt einen am Schaft 12 befestigten Kopf 11, von dem verschiedene Ausführungsformen nachfolgend näher erläutert werden.

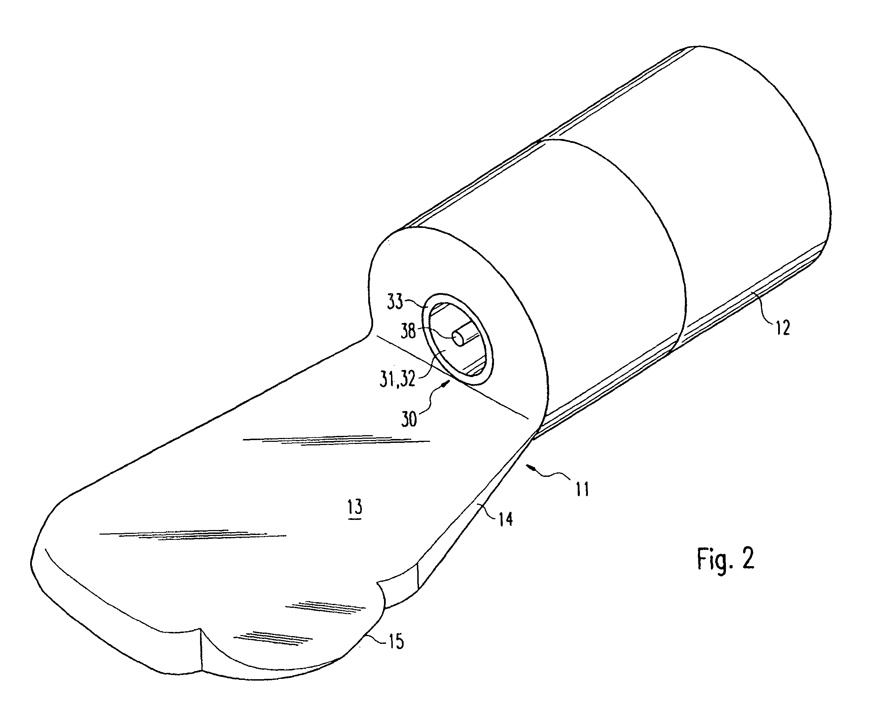

Bei der in Fig. 2 gezeigten Ausführungsform des Kopfes 11 handelt es sich um ein Skalpell-artiges Instrument, das eine Fläche 13 aufweist, deren Rand 14 mit einer Schneide 15 versehen ist.

Im Kopf 11 ist weiterhin eine Koagulationseinrichtung 30 vorgesehen, welche eine Ausströmöffnung 32 einer Gaszuführungseinrichtung 31 umfaßt, die mit einer Gasleitung 33 verbunden ist, durch welche ein ionisierbares Gas, insbesondere ein Edelgas, vorzugsweise Argon oder Helium, zugeführt wird.

Im Inneren der Ausströmöffnung 32 und zwar vorzugsweise derart, daß eine Berührung mit Gewebe ausgeschlossen ist, befindet sich eine Elektrode 38, die mit einer (nicht gezeigten) Stromzuführungseinrichtung verbunden ist. Dieser Elektrode 38 kann ein HF-Koagulationsstrom zugeführt werden, dessen Stromstärke bzw. Spannung in an sich bekannter Weise einstell- bzw. regelbar ist.

Der Kopf 11 des hier gezeigten Arbeitsinstruments 10, natürlich auch die Gasleitung 33 sind nun aus einem elektrisch nicht-leitenden Material hergestellt bzw. mit einem solchen derart überzogen, daß ein durch die Elektrode 38 zugeführter Koagulationsstrom nicht durch den Kopf 11 gestört wird, sondern ausschließlich - über das sich in bekannter Weise bildende Plasma - zum Gewebe fließen kann, bei welchem sich der Kopf 11 des Arbeitsinstruments befindet.

Wenn nun der Operateur während oder direkt nach einem Führen der Schneide 15 in bzw. durch Gewebe den Koagulationsstrom - vorzugsweise mittels eines Fußschalters oder mittels eines am Handgriff 22 angebrachten Schalters - einschaltet, so bildet sich eine Plasmaentladung und der Koagulationsstrom fließt in das Gewebe. Hierbei ist eine Art Selbstregelung derart festzustellen, daß der Koagulationsstrom in erster Linie dort in das Gewebe eintritt, wo es gerade geschnitten und darum besonders feucht ist. Gleiches gilt natürlich auch für Blutgefäße, die - gerade geöffnet - einen besonders niedrigen elektrischen "Oberflächenwiderstand" aufweisen, wodurch der Koagulationseffekt besonders stark auftritt. Mit zunehmender Koagulation bzw. Austrocknung des Gewebes steigt der Widerstand an dieser Stelle an, so daß sich die Entladung eine andere Stelle "sucht" bzw. aufgrund der sich dann ergebenden Widerstandsverhältnisse bei entsprechender HF-Stromversorgung abbricht. Der hier wesentliche Vorteil für den Operateur besteht somit darin, daß im wesentlichen während, zumindest aber sehr kurz nach Führung eines Schnittes sofort koaguliert werden kann, so daß sich Blutungen auf ein Minimum reduzieren lassen. Dies wiederum erleichtert dem Operateur seine Arbeit und schont den Patienten. Dies gilt beispielsweise in besonderem Maße für die Tonsillektomie, bei welcher bekanntermaßen Blutungen ein schwerwiegendes Problem darstellen.

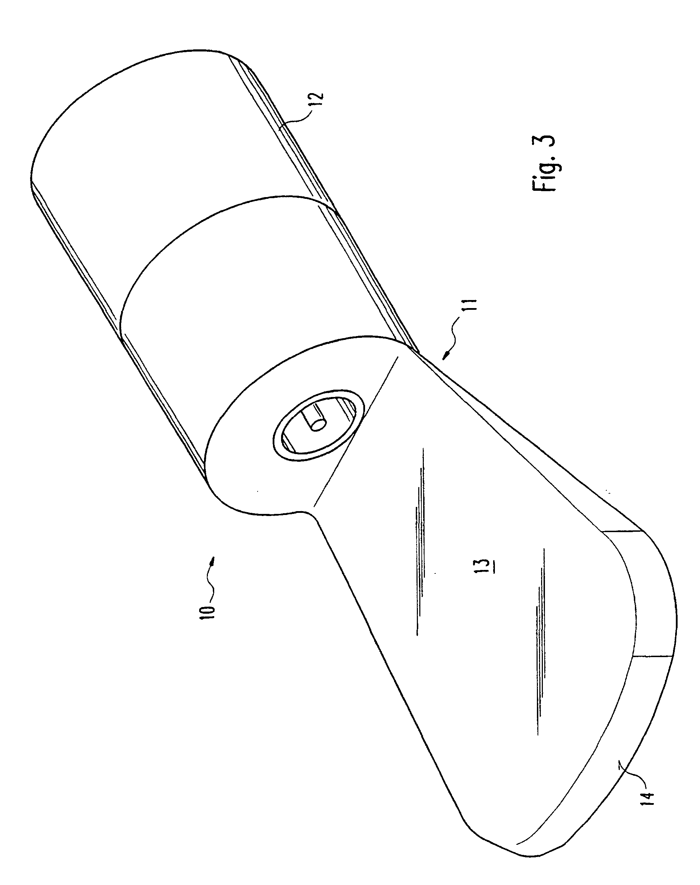

Bei der in Fig. 3 gezeigten Ausführungsform des Kopfes 11 handelt es sich um einen Spatel, der zum Fortschieben von Gewebe gedacht ist.

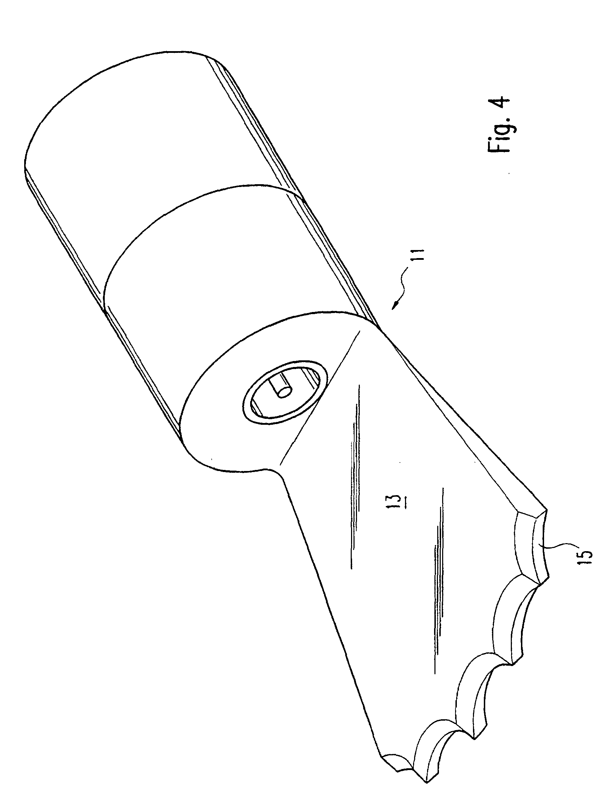

Die in Fig. 4 gezeigte Ausführungsform des Kopfes 11 weist am Vorderrand der Fläche 13 eine mit Zähnen versehene Schneide 15 auf.

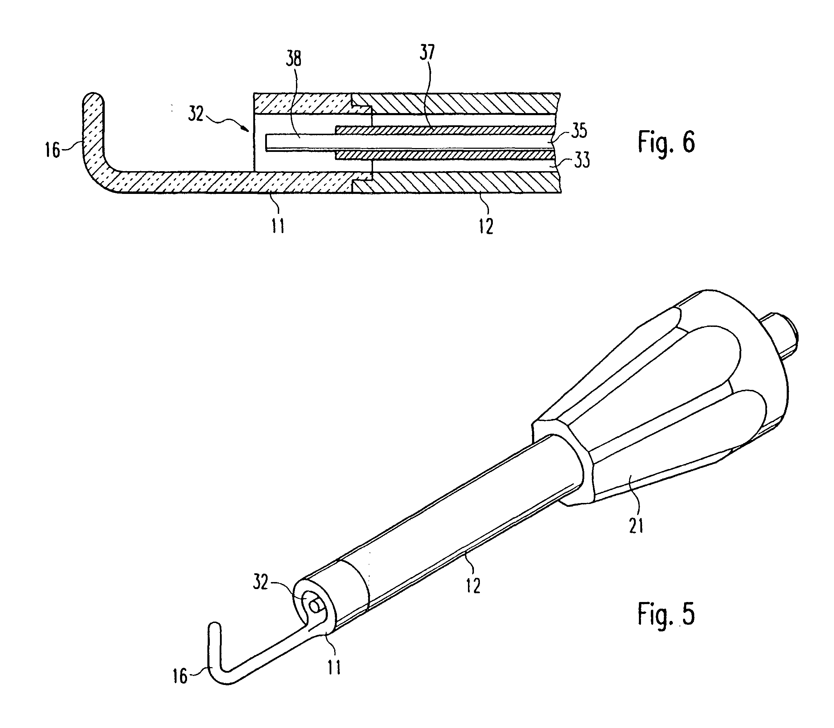

In Fig. 5 ist eine weitere Ausführungsform eines Kopfes 11 gezeigt, der in diesem Fall einen Haken 16 umfaßt. Der Haken 16 ist - wie in Fig. 6 dargestellt - einstückig mit dem Kopf 11 ausgebildet, wobei sich hier - wie auch bei den zuvor und nachfolgend gezeigten Ausführungsbeispielen - elektrisch isolierende und temperaturfeste keramische Werkstoffe besonders eignen.

Der Kopf 11 ist im Schaft 12 befestigt, der in seinem Inneren hohl ist und so eine Gasleitung 33 bildet. Im Schaft 12 bzw. in der Gasleitung 33 ist ein Elektrodenhalter 37 befestigt, durch dessen Inneres ein Draht als Stromzuführungseinrichtung 35 geführt ist, der mit seinem Ende unter Bildung der Elektrode 38 aus dem Elektrodenhalter 37 (unisoliert) hervorsteht. Die Anordnung ist hierbei derart getroffen, daß die Elektrode 38 im wesentlichen konzentrisch innerhalb der Ausströmöffnung 32 sitzt, wobei - wie aus Fig. 6 leicht ersichtlich - die Elektrode 38 etwas zurückgesetzt ist, so daß ein Kontakt zwischen der Elektrode 38 und umgebendem Gewebe nicht möglich ist.

Die Gaszufuhr (durch die Gasleitung 33) wird vorzugsweise so eingestellt, daß ständig, also auch dann, wenn kein Koagulationsstrom fließt, ein gewisser Gasfluß vorhanden ist. Dadurch wird sichergestellt, daß keine Flüssigkeiten in die Ausströmöffnung 32 eindringen und irgendwelche, den Koagulationsstrom störende Wirkung entfalten können.

Es ist nun nicht nur möglich, relativ einfache Präparationsinstrumente, wie in den Fig. 1 bis 6 gezeigt, aus dem elektrisch isolierenden (vorzugsweise keramischen) Material aufzubauen, man kann vielmehr auch sehr viel kompliziertere Instrumente, wie z. B. ein Arbeitsinstrument 10 mit einer Schere 17 herstellen, deren Schneiden 15 sehr nahe bei der Koagulationseinrichtung 30 bzw. bei der Ausströmöffnung 32 mit innenliegender Elektrode 38 angeordnet sind. Durch eine derartige Anordnung ist - nach einem Schnitt - eine sofortige Koagulation des geöffneten Gewebes möglich.

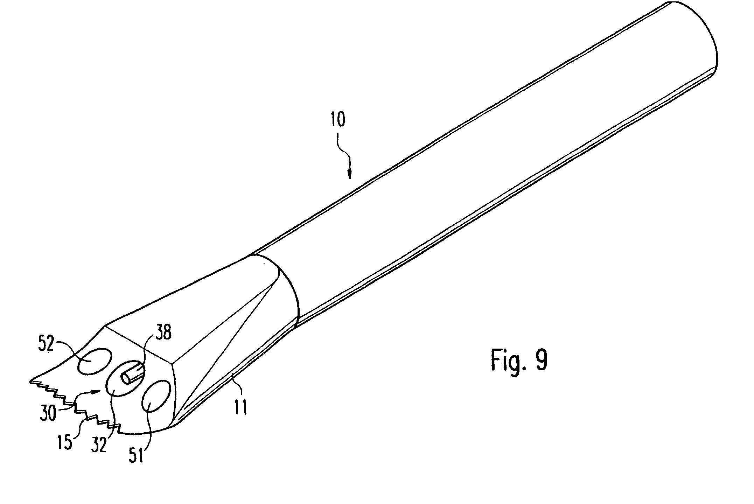

Bei der in Fig. 9 gezeigten weiteren Ausführungsform der erfindungsgemäßen Vorrichtung umfaßt das Arbeitsinstrument 10 bzw. dessen Kopf 11 wieder eine Schneide 15 in der Nähe der Koagulationseinrichtung 30 mit ihrer Gasausströmöffnung 32 mit innenliegender Elektrode 38. Zusätzlich hierzu sind noch zwei Kanäle 51 und 52 vorgesehen, die über entsprechende Leitungen an verschiedenartige Quellen anschließbar sind. Man kann beispielsweise einen Kanal als Saugkanal, den anderen als Spülkanal zum Zuführen einer Flüssigkeit oder auch eines Gases verwenden, um größere Mengen von Flüssigkeit am Operationsfeld abzusaugen bzw. (gegebenenfalls zusammen mit festen Teilchen) fortzuspülen. Wesentlich an dieser Ausführungsform der Erfindung ist die Tatsache, daß auch hier die Zusatzinstrumente, welche bei Fig. 9 als Kanäle ausgebildet sind, im wesentlichen direkt oder unmittelbar bei der Schneide 15 (oder einem entsprechenden Haken usw.) angeordnet sind. Durch diese unmittelbare Nähe kann der Operateur sofort und ohne Wechsel des Instrumentes, im wesentlichen auch ohne größere Verlagerung des Instrumentes zusammen mit bzw. kurz nach Durchführung eines Schnittes (Öffnen des Gewebes mittels eines Hakens oder Spatels usw.) einen weiteren Operationsvorgang, nämlich die genannte Koagulation oder aber ein Fortspülen, Absaugen usw. durchführen. Von der gegenständlichen Ausbildung des Arbeitsinstrumentes 10 her ist die Materialwahl essentiell wichtig, da sichergestellt werden muß, daß der Koagulationsstrom-Fluß von der Elektrode 38 zum hier nicht gezeigten Körpergewebe sichergestellt sein muß.

Die oben beschriebenen Arbeitsinstrumente bzw. verschiedenartigen Köpfe der Arbeitsinstrumente können den Anforderungen des Operateurs entsprechend geformt sein, wobei auch elastische Arbeitsinstrumente, z. B. Schlingen bei entsprechender Materialwahl herstellbar sind. Die gezeigten Instrumente sind nicht auf besondere Anwendungsbereiche beschränkt. Sie können sowohl in der offenen Chirurgie als auch bei endoskopischen Operationen verwendet werden.

### Bezugszeichenliste

- 10: Arbeitsinstrument
- 11: Kopf
- 12: Schaft
- 13: Fläche
- 14: Rand
- 15: Schneide
- 16: Haken
- 17: Schere
- 20: Halteeinrichtung
- 21: Befestigungskonus
- 22: Handgriff
- 30: Koagulationseinrichtung
- 31: Gaszuführungseinrichtung
- 32: Ausströmöffnung
- 33: Gasleitung
- 37: Elektrodenhalter
- 38: Elektrode
- 40: Anschlußschlauch
- 51: Saugkanal
- 52: Spülkanal

## Patentansprüche

1. Präparierinstrument für chirurgische Operationen, umfassend
mindestens ein Arbeitsinstrument (10) zum Bearbeiten von Gewebe; eine Halteeinrichtung (20), z.B. einen Handgriff, Manipulator, zum Befestigen und Bewegen des Arbeitsinstruments (10);
eine Koagulationseinrichtung, umfassend
- eine Gaszuführungseinrichtung (31) zum Zuführen eines ionisierbaren Gases, insbesondere eines Edelgases zu einem Bereich bei dem Gewebe;
- eine Stromzuführungseinrichtung (35) zum Zuführen eines Koagulationsstroms von einer Elektrodeneinrichtung (36) zum Gewebe;
- eine steuerbare Stromquelle, insbesondere eine HF-Quelle zum Erzeugen des durch Plasma geleiteten Koagulationsstroms von der Elektrodeneinrichtung (36) zum Gewebe und zum Koagulieren, insbesondere zum Blutstillen;
wobei die Elektrodeneinrichtung (36) getrennt vom Arbeitsinstrument (10) angeordnet ist, wobei
das Arbeitsinstrument (10) eine Schneide oder ein Messer oder eine Schere oder einen Haken oder eine Nadel oder eine Schlinge zum Schneiden oder Öffnen oder Schieben oder Ziehen von Gewebe ist und wobei
das Arbeitsinstrument derart aus einem isolierenden Material ausgebildet oder mit einem isolierenden Material überzogen und distal von der Elektrodeneinrichtung (36) derart angeordnet ist, dass der Koagulationsstrom auch während einer Gewebeberührung durch das Arbeitsinstrument (10), ohne durch dieses geleitet zu werden, dem Gewebe zuführbar ist.

2. Präparierinstrument nach einem der vorhergehenden Ansprüche,
**dadurch gekennzeichnet, dass**
die Elektrodeneinrichtung einen Elektrodenhalter (37), und eine Elektrode (38), insbesondere eine Drahtelektrode umfasst und nahe bei dem Arbeitsinstrument (10) mit diesem zusammen fixiert ist.

3. Präparierinstrument nach einem der vorhergehenden Ansprüche,
**dadurch gekennzeichnet, dass**
die Gaszuführungseinrichtung eine Ausströmöffnung (32) einer Gasleitung (33) umfasst, die bei der Elektrodeneinrichtung (36) oder diese umgebend nahe dem Arbeitsinstrument (10) angeordnet ist.

4. Präparierinstrument nach Anspruch 2,
**dadurch gekennzeichnet, dass**
das Arbeitsinstrument (10) und der Elektrodenhalter (37) einstückig ausgebildet sind.

5. Präparierinstrument nach Anspruch 3,
**dadurch gekennzeichnet, dass**
das Arbeitsinstrument (10) und die Gasausströmöffnung (32) einstückig ausgebildet sind.

6. Präparierinstrument nach einem der vorhergehenden Ansprüche,
**dadurch gekennzeichnet, dass**
das Arbeitsinstrument (10) und mindestens ein distaler Endabschnitt der Halteeinrichtung (20) einstückig ausgebildet sind.

7. Präparierinstrument nach einem der Ansprüche 2-6,
**dadurch gekennzeichnet, dass**
die Elektrode (38) derart mit einem Schutzkorb versehen oder in einer Gasausströmöffnung (32) angeordnet ist, dass ein direkter elektrischer Kontakt zwischen der Elektrodeneinrichtung (37, 38) und dem Gewebe verhindert wird.

8. Präparierinstrument nach einem der vorhergehenden Ansprüche,
**gekennzeichnet durch**
ein beim Arbeitsinstrument (10) angebrachtes Zusatzinstrument (51, 52), insbesondere eine Saug-, Spül-, Blaseinrichtung, ein zusätzliches elektrochirurgisches Instrument, wie Nadel-, Kugel-, Draht-, Schlingen- oder Flach-Elektrode.

9. Präparierinstrument nach einem der vorhergehenden Ansprüche,
**dadurch gekennzeichnet, dass**
die Halteeinrichtung (20) auswechselbar unter gleichzeitiger An- und Abkopplung von Gas- und Stromzufuhr ausgebildet ist.

10. Präparierinstrument nach einem der vorhergehenden Ansprüche,
**dadurch gekennzeichnet, dass**
Reinigungseinrichtungen zum Freihalten der Elektrodeneinrichtung (38) von Flüssigkeit oder dergleichen verschmutzenden Substanzen vorgesehen sind.

11. Präparierinstrument nach Anspruch 12,
**dadurch gekennzeichnet, dass**
die Reinigungseinrichtungen eine gesonderte Gasleitungseinrichtung oder die Gaszuführungseinrichtung (31) umfassen, die derart ausgebildet ist, dass die Elektrodeneinrichtung (38) von einem im Wesentlichen ständigen Gasstrom umspülbar ist.

## Claims

1. Dissecting instrument for surgical operations, comprising
at least one working instrument (10) for working on tissue; a holding device (20), e.g.
a handle, manipulator, for attaching and moving the working instrument (10);
a coagulation device, comprising
- a gas supply device (31) for supplying an ionizable gas, in particular an inert gas, to an area in the tissue;
- a current supply device (35) for supplying a coagulation current from an electrode device (36) to the tissue;
- a controllable current source, in particular a HF source, for generating the coagulation current conducted through plasma from the electrode device (36) to the tissue and for coagulation, in particular for blood staunching;
wherein the electrode device (36) is disposed separately from the working instrument (10), wherein
the working instrument (10) is a cutting edge or a knife or scissors or a hook or a needle or a loop for cutting or opening or pushing or pulling tissue, and wherein
the working instrument is formed from an insulating material or is covered with an insulating material and disposed distally from the electrode device (36) such that the coagulation current can also be supplied to the tissue during tissue contact through the working instrument (10) without being conducted through this.

2. Dissecting instrument according to any one of the preceding Claims,
**characterised in that**
the electrode device comprises an electrode holder (37) and an electrode (38), in particular a wire electrode, and is fixed together with the working instrument (10) near the latter.

3. Dissecting instrument according to any one of the preceding Claims,
**characterised in that**
the gas supply device comprises an outflow opening (32) of a gas conduit (33) which is disposed at the electrode device (36) or, surrounding the latter, near the working instrument (10).

4. Dissecting instrument according to Claim 2,
**characterised in that**
the working instrument (10) and the electrode holder (37) are formed in one piece.

5. Dissecting instrument according to Claim 3,
**characterised in that**
the working instrument (10) and the gas outflow opening (32) are formed in one piece.

6. Dissecting instrument according to any one of the preceding Claims,
**characterised in that**
the working instrument (10) and at least one distal end portion of the holding device (20) are formed in one piece.

7. Dissecting instrument according to any one of Claims 2-6,
**characterised in that**
the electrode (38) is provided with a protective cage or disposed in a gas outflow opening (32) such that direct electrical contact between the electrode device (37, 38) and the tissue is prevented.

8. Dissecting instrument according to any one of the preceding Claims,
**characterised by** an auxiliary instrument (51, 52), in particular a suction, irrigating, blowing device, which is fitted at the working device (10), an additional electrosurgical instrument such as a needle, ball, wire, loop or flat electrode.

9. Dissecting instrument according to any one of the preceding Claims,
**characterised in that**
the holding device (20) is formed so as to be interchangeable, with simultaneous connection and disconnection of gas and current supply.

10. Dissecting instrument according to any one of the preceding Claims,
**characterised in that**
cleaning devices for keeping the electrode device (38) free of fluid or contaminating substances of this kind are provided.

11. Dissecting instrument according to Claim 12,
**characterised in that**
the cleaning devices comprise a separate gas conduction device or the gas supply device (31) which is formed such that a substantially continuous gas stream can wash around the electrode device (38).

## Revendications

1. Instrument de préparation pour opérations chirurgicales, comprenant
au moins un instrument de travail (10) pour traiter un tissu ;
un dispositif de retenue (20), par exemple une poignée, un manipulateur, pour fixer et déplacer l'instrument de travail (10) ;
un dispositif de coagulation, comprenant
- un dispositif d'amenée de gaz (31) pour amener un gaz ionisable, en particulier un gaz rare, à une zone dans le tissu ;
- un dispositif d'amenée de courant (35) pour amener un courant de coagulation d'un dispositif à électrode (36) au tissu ;
- une source de courant réglable, en particulier une source HF pour générer le courant de coagulation conduit à travers le plasma, du dispositif à électrode (36) au tissu, et pour coaguler, en particulier pour arrêter l'hémorragie ;
dans lequel le dispositif à électrode (36) est disposé séparément de l'instrument de travail (10),
dans lequel l'instrument de travail (10) est à une lame ou un couteau ou des ciseaux ou un crochet ou une aiguille ou une boucle pour couper ou ouvrir ou pousser ou tirer un tissu, et
dans lequel l'instrument de travail est réalisé à partir d'un matériau isolant ou est recouvert d'un matériau isolant et disposé de façon distale par rapport au dispositif à électrode (36) de telle sorte que le courant de coagulation peut être amené au tissu, même lorsque l'instrument de travail (10) est en contact avec le tissu, sans être conduit à travers celui-ci.

2. Instrument de préparation selon l'une quelconque des revendications précédentes, **caractérisé en ce que** le dispositif à électrode comprend un porte-électrode (37) et une électrode (38), en particulier une électrode en fil, et est fixé à l'instrument de travail (10) à proximité de celui-ci.

3. Instrument de préparation selon l'une quelconque des revendications précédentes, **caractérisé en ce que** le dispositif d'amenée de gaz comprend un orifice d'évacuation (32) d'une conduite de gaz (33) qui est disposée au niveau du dispositif à électrode (36), ou en contournant celui-ci, à proximité de l'instrument de travail (10).

4. Instrument de préparation selon la revendication 2, **caractérisé en ce que** l'instrument de travail (10) et le porte-électrode (37) sont réalisés d'une seule pièce.

5. Instrument de préparation selon la revendication 3, **caractérisé en ce que** l'instrument de travail (10) et l'orifice d'évacuation de gaz (32) sont réalisés d'une seule pièce.

6. Instrument de préparation selon l'une quelconque des revendications précédentes, **caractérisé en ce que** l'instrument de travail (10) et au moins une section d'extrémité distale du dispositif de retenue (20) sont réalisés d'une seule pièce.

7. Instrument de préparation selon l'une quelconque des revendications 2 à 6, **caractérisé en ce que** l'électrode (38) est munie d'une cage protectrice ou est disposée dans un orifice d'évacuation de gaz (32) de telle sorte qu'un contact électrique direct est empêché entre le dispositif à électrode (37, 38) et le tissu.

8. Instrument de préparation selon l'une quelconque des revendications précédentes, **caractérisé par** un instrument supplémentaire (51, 52) installé au niveau de l'instrument de travail (10), en particulier un dispositif d'aspiration, un dispositif de rinçage, un dispositif de soufflage, un instrument électrochirurgical supplémentaire, comme une électrode à aiguille, une électrode à bille, une électrode en fil, une électrode en boucle ou une électrode plate.

9. Instrument de préparation selon l'une quelconque des revendications précédentes, **caractérisé en ce que** le dispositif de retenue (20) est réalisé de façon à pouvoir être échangé avec un couplage et découplage simultanés de l'amenée de gaz et de courant.

10. Instrument de préparation selon l'une quelconque des revendications précédentes, **caractérisé en ce que** des dispositifs de nettoyage pour maintenir le dispositif à électrode (38) exempt de tout liquide ou de substances polluantes de ce type sont prévus.

11. Instrument de préparation selon la revendication 12, **caractérisé en ce que** les dispositifs de nettoyage comprennent un dispositif de conduite de gaz séparée ou le dispositif d'amenée de gaz (31) qui est réalisé de telle sorte que le dispositif à électrode (38) peut être balayé par un flux de gaz substantiellement continu.
